(19) Europäisches Patentamt
European Patent Office
Office européen des brevets

(11) EP 3 127 895 A1

(12) EUROPEAN PATENT APPLICATION
published in accordance with Art. 153(4) EPC

(43) Date of publication:
08.02.2017 Bulletin 2017/06

(51) Int Cl.:
C07C 227/44 (2006.01)        C07C 229/22 (2006.01)
G01N 27/62 (2006.01)         G01N 33/50 (2006.01)

(21) Application number: 15774050.7

(22) Date of filing: 31.03.2015

(86) International application number:
PCT/JP2015/060110

(87) International publication number:
WO 2015/152234 (08.10.2015 Gazette 2015/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA

(30) Priority: 31.03.2014  JP 2014074163
15.12.2014  JP 2014252820

(71) Applicant: Sekisui Medical Co., Ltd.
Tokyo 103-0027 (JP)

(72) Inventors:
• SHIROSHITA, Tomoyoshi
Tokyo 103-0027 (JP)
• OH-YAMA, Naoko
Tokyo 103-0027 (JP)
• IDENO, Akira
Tokyo 103-0027 (JP)
• OH-HARA, Toshinari
Tokyo 103-0027 (JP)

(74) Representative: Hoffmann Eitle
Patent- und Rechtsanwälte PartmbB
Arabellastraße 30
81925 München (DE)

(54) **ACYLCARNITINE COMPOSITION**

(57)    A problem to be solved by the present invention is to provide an acylcarnitine composition easy to handle in a solution state and having long-term storage stability. The problem is solved by an acylcarnitine composition characterized by having at least one or more acylcarnitines dissolved in an organic solvent containing an organic acid.

**Description**

TECHNICAL FIELD

The present invention relates to an acylcarnitine composition. The present invention more particularly relates to a composition enabling long-term stable storage of acylcarnitine.

BACKGROUND ART

Acylcarnitine is a compound generated by binding of a fatty acid and carnitine when a fatty acid is transported to a mitochondrial inner membrane in a living body. More particularly, acylcarnitine is generated from acyl-CoA and carnitine due to the action of carnitine palmitoyltransferase I present in the mitochondrial outer membrane.

Acylcarnitine is finely classified into acetylcarnitine, propionylcarnitine, stearoylcarnitine, oleylcarnitine, linoleylcarnitine, malonylcarnitine, 3-hydroxycarnitine, and others, based on the carbon chain length of the fatty acid portion thereof, the presence and number of unsaturated bonds, the substitution of a hydrogen atom coupled to a carbon chain to an oxygen atom or a hydroxy group, and the like. These acylcarnitines are collectively referred to as acylcarnitine in this description. In this description, free carnitine without a fatty acid bonded thereto is also included in acylcarnitine.

Particularly, it is extremely valuable in the medical field to analyze acylcarnitine in a biological sample such as blood and urine so as to evaluate metabolic disorder, and thus, acylcarnitine is an item to be analyzed in the screening of newborn babies for inborn errors of metabolism, for example.

It is described in Patent Document 1 that a profile of acylcarnitine acquired by measuring acylcarnitine in plasma by using a mass spectrometer can be utilized for the diagnosis of fatty acid oxidation disorders (MCAD, VLCAD, SCAD, MAD, LCHAD, and CPTII) as well as some of organic acidemias (such as propionic acidemia, methylmalonic acidemia, isovaleric acidemia, glutaric acidemia type 1, 3-methylcrotonyl-CoA carboxylase deficiency, and β-ketothiolase deficiency).

In general, when acylcarnitine is quantitatively measured by using a mass spectrometer, a method may employ acylcarnitine as a standard substance or isotope-labeled acylcarnitine as an internal standard substance. These standard and internal standard substances can be acquired by purchasing individually from reagent manufacturers or by purchasing a kit such as a NeoBase Non-Derivatized MSMS kit (manufactured by PerkinElmer) described in Non-Patent Document 1. However, these standard and internal standard substances are freeze-dried products and require a step of completely dissolving by adding a suitable solution before use.

CITATION LIST

PATENT LITERATURE

Patent Document 1: Japanese Patent No. 4838129

NON PATENT LITERATURE

Non-Patent Document 1: "3040-0010 NeoBase Non-derivatized MSMS kit Instruction Manual (PerkinElmer)"
Non-Patent Document 2: "Stability of Acylcarnitine in Liquid Sample," Health and Labour Sciences Research Grants in FY 2011 (Basic Research Project for Promotion of Next Generation for Overcoming Developmental Disease, etc.), "Study on the Development and Qualitative Improvement of Mass-Screening System for Newborns through Introduction of Tandem Mass," General/Allotted Research Report in FY 2011, Seiji Yamaguchi et al., pp40-43, Ministry of Health, Labor and Welfare, 2012

SUMMARY OF INVENTION

TECHNICAL PROBLEM

For generally eliminating the step of dissolving a standard substance and an internal standard substance, reagents prepared as solution in advance are commercially available from several manufactures. For example, Wako Pure Chemical Industries, Ltd. sells amino acid solutions such as a 200 mmol/L L-glutamine solution (×100) and a MEM non-essential amino acid solution (×100).

However, as described in Non-Patent Document 1, the stability of acylcarnitine in a solution state is 30 days at 2 °C to 8 °C, and it is described that the acylcarnitine solution does not have storage stability for a period exceeding one month.

In Non-Patent Document 2, the stability of acylcarnitine solution is studied in more detail. Since degradation of long-chain acylcarnitines was detected in a buffer solution of pH 8.0 stored in a refrigerator (4 °C), and degradation of various acylcarnitines from short-chain to long-chain was detected in serum of healthy persons stored at normal temperature (21 °C), frozen storage (-30 °C) is recommended for all the specimens (blood on filter paper, serum, urine) used in acylcarnitine analysis.

In particular, an acylcarnitine solution is not commercially available because of the problem of storage stability of acylcarnitine in a solution state. Therefore, a demand from measurement practitioners exists for an acylcarnitine composition that is utilizable as a reagent for a standard substance and a reagent for internal standard, that eliminates the need for a work of dissolving acylcarnitine, and that has long-term storage stability.

From the above, a problem to be solved by the present invention is to provide an acylcarnitine composition easy to handle in a solution state and having long-term storage stability.

SOLUTION TO PROBLEM

The present inventors found that acylcarnitine can stably be stored for a long term in a solution state by dissolving acylcarnitine in an organic solvent containing an organic acid, thereby completing the present invention.

In Non-Patent Document 2, it is suggested that in neutral buffers of pH 6.0 to 8.0, hydrolysis of acylcarnitine increases at pH 8.0; however, it was not found out that acylcarnitine was stable in an acidic aqueous solution. Moreover, the stability of acylcarnitine in an organic solvent is not mentioned.

The present inventors have discovered that the storage stability of acylcarnitine in solution is dramatically improved by dissolving acylcarnitine in an organic solvent and adding an organic acid. Additionally, the present inventors have completed an acylcarnitine composition not affecting mass spectrometers by using organic acids generally used in mass spectrometry.

The present invention is related to the following details.

<1> An acylcarnitine composition, wherein
at least one or more compounds of Formula 1 are dissolved in an organic solvent containing an organic acid:

$$\text{Formula 1:} \qquad (CH_3)_3N^+CH_2CH(OR^1)CH_2COO^-$$

wherein in Formula 1, $R^1$ denotes a hydrogen atom, or a saturated or unsaturated fatty acid residue having a carbon number of 2 to 30, and wherein the hydrogen atom(s) bonded to the fatty acid residue may be substituted with an oxygen atom(s) or a hydroxy group(s).

<2> The acylcarnitine composition according to <1>, wherein the organic solvent is an organic solvent having a carbon number of 1 to 5.

<3> The acylcarnitine composition according to <1> or <2>, wherein the organic solvent is at least one or more selected from the group consisting of alcohol having a carbon number of 1 to 5 and acetonitrile.

<4> The acylcarnitine composition according to <3>, wherein the alcohol having a carbon number of 1 to 5 is at least one or more selected from the group consisting of methanol, ethanol, and isopropanol.

<5> The acylcarnitine composition according to any one of <1> to <4>, wherein the organic acid is at least one or more selected from the group consisting of monovalent to trivalent carboxylic acids.

<6> The acylcarnitine composition according to <5>, wherein the carboxylic acid is at least one or more selected from the group consisting of formic acid, acetic acid, oxalic acid, trifluoroacetic acid, citric acid, butyric acid, and lactic acid.

<7> The acylcarnitine composition according to any one of <1> to <6>, wherein the volumetric concentration of the organic acid is 0.005 %(v/v) to 5 %(v/v).

<8> The acylcarnitine composition according to any one of <1> to <7>, wherein acylcarnitine is isotope-labeled.

<9> The acylcarnitine composition according to any one of <1> to <8>, wherein when the acylcarnitine composition is stored at 45 °C for 24 hours, the residual rate of acylcarnitine calculated from the value of measurement with a mass spectrometer is 80 % to 120 %.

<10> A method of measuring acylcarnitine with a mass spectrometer, the method comprising: using the acylcarnitine composition according to any one of <1> to <9>.

<11> A use of the acylcarnitine composition according to any one of <1> to <9> in a diagnosis and screening for inborn errors of metabolism with a mass spectrometer.

<12> A kit comprising the acylcarnitine composition according to any one of <1> to <9>.

<13> A kit comprising: an organic acid, an organic solvent, and at least one or more acylcarnitine compounds of Formula 1:

Formula 1 : $(CH_3)_3N^+CH_2CH(OR^1)CH_2COO^-$

wherein in Formula 1, $R^1$ denotes a hydrogen atom, or a saturated or unsaturated fatty acid residue having a carbon number of 2 to 30, and wherein the hydrogen atom(s) bonded to the fatty acid residue may be substituted with an oxygen atom(s) or a hydroxy group(s).

ADVANTAGEOUS EFFECTS OF INVENTION

The present invention provides an acylcarnitine composition having long-term storage stability without the need for a work of dissolving acylcarnitine. The acylcarnitine composition of the present invention is useful for clinical examination such as a screening for inborn errors of metabolism.

BRIEF DESCRIPTION OF DRAWINGS

[Fig. 1] Fig. 1 is a plot for prediction of stability of acetylcarnitine (C2)-stable isotope in methanol acquired in Reference Example 1.
[Fig. 2] Fig. 2 is a histogram for stability of acetylcarnitine (C2)-stable isotope in methanol containing 0.1 % formic acid and various organic solvents acquired in Example 1.
[Fig. 3] Fig. 3 is a histogram for stability of acetylcarnitine (C2)-stable isotope in methanol containing 1 % formic acid and various organic solvents acquired in Example 2.
[Fig. 4] Fig. 4 is a histogram for stability of acylcarnitine (C0 to C18)-stable isotopes in methanol and ethanol containing 1 % formic acid acquired in Example 3.
[Fig. 5] Fig. 5 is a histogram for stability of acetylcarnitine (C2)-stable isotope in acetonitrile containing 1 % formic acid and various organic solvents acquired in Example 4.
[Fig. 6] Fig. 6 is a histogram for stability of acetylcarnitine (C2)-stable isotope in ethanol containing formic acid at indicated concentrations acquired in Example 5.
[Fig. 7] Fig. 7 is a histogram for stability of acetylcarnitine (C2)-stable isotope in ethanol containing acetic acid at indicated concentrations acquired in Example 6.
[Fig. 8] Fig. 8 is a histogram for stability of acetylcarnitine (C2)-stable isotope in ethanol containing lactic acid at indicated concentrations acquired in Example 7.
[Fig. 9] Fig. 9 is a histogram for stability of acetylcarnitine (C2)-stable isotope in ethanol containing oxalic acid at indicated concentrations acquired in Example 8.
[Fig. 10] Fig. 10 is a histogram for stability of acetylcarnitine (C2)-stable isotope in ethanol containing citric acid at indicated concentrations acquired in Example 9.

DESCRIPTION OF EMBODIMENTS

In the present invention, acylcarnitine refers to compounds represented by Formula 1:

Formula 1 : $(CH_3)_3N^+CH_2CH(OR^1)CH_2COO^-$

(in Formula 1, $R^1$ denotes hydrogen atom, or a saturated or unsaturated fatty acid residue having a carbon number of 2 to 30, and a hydrogen atom(s) bonded to the fatty acid residue may be substituted with an oxygen atom(s) or a hydroxy group(s)).

The saturated or unsaturated fatty acid residue may be those described in Table 1 and, among them, fatty acid residues having a carbon number of 2 to 18 are preferably used. Major acylcarnitines used for an item of clinical evaluation such as a screening for inborn errors of metabolism are listed in Table 2.

[Table 1]

| Compound Name | Code |
|---|---|
| Formic Acid | 1:0 |
| Acetic Acid | 2:0 |

(continued)

| Compound Name | Code |
|---|---|
| Propionic Acid | 3:0 |
| Butyric Acid | 4:0 |
| Valeric Acid | 5:0 |
| Caproic Acid | 6:0 |
| Enanthic Acid | 7:0 |
| Caprylic Acid | 8:0 |
| Pelargonic Acid | 9:0 |
| Capric Acid | 10:0 |
| Lauric Acid | 12:0 |
| Myristic Acid | 14:0 |
| Pentadecylic Acid | 15:0 |
| Palmitic Acid | 16:0 |
| Palmitoleic Acid | 16:1 |
| Margaric Acid | 17:0 |
| Stearic Acid | 18:0 |
| Oleic Acid | 18:1 (9) |
| Vaccenic Acid | 18:1 (11) |
| Linoleic Acid | 18:2 (9, 12) |
| (9,12,15)-Linotenic Acid | 18:3 (9, 12, 15) |
| (6,9,12)-Linolenic Acid | 18:3(6, 9, 12) |
| Eleostearic Acid | 18:3(9, 11, 13) |
| Arachidic Acid | 20:0 |
| 8,11-Eicosadienoic Acid | 20:2 (8, 11) |
| 5,8,11-Eicosatrienoic Acid | 20:3 (5, 8, 11) |
| Arachidonic Acid | 20:4 (5, 8, 11, 14) |
| Eicosapentaenoic Acid | 20:5 |
| Behenic Acid | 22:0 |
| Docosahexaenoic Acid | 22:6 |
| Lignoceric Acid | 24:0 |
| Nervonic Acid | 24:1 |
| Cerotic Acid | 26:0 |
| Montanoic Acid | 28:0 |
| Melissic Acid | 30:0 |

[Table 2]

| Compound Name | Code |
|---|---|
| Free Carnitine | C0 |

(continued)

| Compound Name | Code |
|---|---|
| Acetylcarnitine | C2 |
| Propionylcarnitine | C3 |
| Malonylcarnitine | C3DC |
| Butyrylcarnitine | C4 |
| 3-Hydroxy-Butyrylcarnitine | C4OH |
| Isovalerylcarnitine | C5 |
| Tiglylcarnitine | C5:1 |
| Glutarylcarnitine | C5DC |
| 3-Hydroxy-Isovalerylcarnitine | C5OH |
| Hexanoylcarnitine | C6 |
| Adipylcarnitine | C6DC |
| Octanoylcarnitine | C8 |
| Octenoylcarnitine | C8 : 1 |
| Decanoylcarnitine | C10 |
| Decenoylcarnitine | C10 : 1 |
| Decadienoylcarnitine | C10 : 2 |
| Dodecanoylcarnitine (Lauroylcarnitine) | C12 |
| Dodecenoylcarnitine | C12 : 1 |
| Tetradecanoylcarnitine (Myristoylcarnitine) | C14 |
| Tetradecenoylcarnitine | C14 : 1 |
| Tetradecadienoylcarnitine | C14 : 2 |
| 3-Hydroxy-Tetradecanoylcarnitine | C14OH |
| Hexadecanoylcarnitine (Palmitoylcarnitine) | C16 |
| Hexadecenoylcarnitine | C16 : 1 |
| 3-Hydroxy-Hexadecanoylcarnitine | C16OH |
| 3-Hydroxy-Hexadecenoylcarnitine | C16 : 1OH |
| Octadecanoylcarnitine (Stearoylcarnitine) | C18 |
| Octadecenoylcarnitine (Oleylcarnitine) | C18 : 1 |
| Octadecadienoylcarnitine (Linoleoylcarnitine) | C18 : 2 |
| 3-Hydroxy-Octadecanoylcarnitine | C18OH |
| 3-Hydroxy-Octadecenoylcarnitine | C18 : 1OH |

In this description, the term "acylcarnitine" includes an isotope-labeled acylcarnitine. According to the present invention, an isotope-labeled acylcarnitine has the same storage stability. For example, acylcarnitine labeled with a stable isotope (hereinafter referred to as an acylcarnitine-stable isotope) is exemplified by $^2H9$-carnitine, $^2H3$-acetylcarnitine, $^2H3$-propionylcarnitine, $^2H3$-butyrylcarnitine, $^2H9$-isovalerylcarnitine, $^2H3$-octanoylcarnitine, $^2H9$-myristoylcarnitine, and $^2H3$-palmitoylcarnitine. Isotope labeling may be labeling with a radioisotope or labeling with other elements such as carbon, nitrogen, and oxygen, and is not limited to deuterium labeling although deuterium labeling is presented as examples of isotope labeling. Moreover, the number of substituted atoms and the positions of substitutions are not limited to those of the exemplified acylcarnitine-stable isotopes.

An organic solvent used in the present invention is not particularly limited as long as the organic solvent is generally used in biochemistry. Because of the need for efficiently dissolving a mixture of acylcarnitines different in the carbon number, the easiness to handle, and the availability, the organic solvent is preferably an organic solvent having a carbon number of 1 to 5, more preferably alcohol having a carbon number of 1 to 5 and acetonitrile, and further preferably methanol, ethanol, and isopropanol. Since acylcarnitine can be hydrolyzed, the solvent preferably does not contain water.

The organic solvent of the present invention includes an organic acid as in the case described later.

A concentration range of acylcarnitine dissolved in an organic solvent is not particularly limited and may have preferably a lower limit of 1 μmol/L and preferably an upper limit of 100 μmol/L, and the upper limit may preferably be 70 μmol/L, 50 μmol/L, 40 μmol/L, or 35 μmol/L in some cases. If the concentration of acylcarnitine is less than 1 μmol/L, chemical degradation proceeds and the stability in the case of long-term storage is adversely affected. If the concentration of acylcarnitine exceeds 100 μmol/L, acylcarnitine must be diluted multiple times to a concentration suitable for measurement when subjected to mass spectrometry, which makes experimental operations cumbersome.

Solubility of free carnitine in methanol is at most approx. 0.4 g/mL (approx. 2.48 mol/L) and free carnitine is known to be easily soluble in alcohol.

An organic acid used in the present invention is not particularly limited as long as the organic acid is generally used in biochemistry. Because of the easiness to handle and the availability, the organic acid is preferably a monovalent to trivalent carboxylic acid and can be exemplified by formic acid, acetic acid, oxalic acid, trifluoroacetic acid, citric acid, butyric acid, and lactic acid. Formic acid, acetic acid, oxalic acid, and trifluoroacetic acid are preferable from the viewpoint of volatility required for measurement with a mass spectrometer, and formic acid, acetic acid, and oxalic acid are more preferable from the viewpoint of corrosivity.

Although a concentration range of the organic acid is not particularly limited, the concentration range in volumetric concentration has a preferable lower limit of 0.005 %(v/v), while the preferable lower limit may be 0.01 %(v/v), 0.15 %(v/v), 0.2 %(v/v), 0.25 %(v/v), 0.3 %(v/v), 0.35 %(v/v), 0.4 %(v/v), or 0.45 %(v/v) in some cases, and a more preferable lower limit is 0.5 %(v/v).

The concentration range has a preferable upper limit of 5 %(v/v), while the preferable upper limit may be 4.5 %(v/v), 4.0 %(v/v), or 3.5 %(v/v) in some cases, and a more preferable upper limit is 3 %(v/v).

If the concentration of the organic acid is lower than the lower limit, an improvement is not expected in the storage stability of an acylcarnitine composition. If the concentration of the organic acid is higher than the upper limit, the possibility of the organic acid adversely affecting devices and measurement results remains when measurement with a mass spectrometer is performed according to the present invention.

The acylcarnitine composition according to the present invention can preferably be used in acylcarnitine measurement using a mass spectrometer as a standard substance, a calibrator, a control, an internal standard substance, etc.

In an embodiment of the present invention, acylcarnitine, the organic acid, and the organic solvent are preliminarily mixed in a solution state and supplied.

In another embodiment of the present invention, each of the components are separately prepared and mixed in a solution state when used, or two of the components preliminarily mixed and the remaining one component separately prepared are mixed in a solution state when used

In another embodiment of the present invention, a composition which an organic solvent may be added to afterwards may be prepared. For example, a mixture of acylcarnitine and an organic acid is mixed with an organic solvent, a porous member or an excipient capable of retaining organic acids is added to the mixture, and then, the organic solvent is removed to obtain the composition. Alternatively, acylcarnitine, an organic acid, and an organic solvent are mixed, and then, the organic solvent is removed to obtain the composition.

In another embodiment of the present invention, an organic acid and an organic solvent are added to freeze-dried acylcarnitine, or a mixture of an organic acid and an organic solvent is added to acylcarnitine contained in a porous member.

In another embodiment of the present invention, acylcarnitine and an organic solvent are mixed and subsequently an organic acid is added, or an organic solvent and an organic acid are mixed in advance and subsequently added to acylcarnitine.

A porous member can be used without limitation if the stability of acylcarnitine is not impaired when stored at room temperature, and if the porous member is insoluble to a sample extraction liquid and has abilities to elute acylcarnitine into a solution and to retain acylcarnitine in a dry state.

The porous member can be a known porous member and may be a porous member made of, for example, filter paper, nonwoven fabric, woven fabric, sheet foam, or the like. With regard to the material, known natural and synthetic polymers are usable, for example, cotton, sheep wool, cellulose, polystyrene, polyolefin, polyurethane, nitrocellulose, cellulose acetate, polyester, epoxy resin, phenol resin, silk, fibroin, lignin, hemicellulose, chitin, ebonite, rubber, glass, quartz, ceramics, and others. Among them, the material is preferably natural polymers, more preferably cellulose and cotton, and particularly preferably cellulose.

The acylcarnitine composition of the present invention can be used for a screening for inborn errors of metabolism by the acylcarnitine measurement using a mass spectrometer.

The acylcarnitine composition of the present invention can be provided in a form of a measurement kit including an organic acid, an organic solvent, and at least one or more acylcarnitine compounds, and the kit can additionally include other assay reagents, a specimen diluent, an instruction manual, and others. The other assay reagents may be an assay reagent for amino acids or succinylacetone expected to be measured at the same time and, specifically, the reagents may be internal standard solutions thereof.

[Reference Example 1] Stability Prediction of Acetylcarnitine (C2) in Methanol

First, to find less stable acylcarnitine out of acylcarnitines, residual rates of acylcarnitine-stable isotopes listed in Table 3 were calculated in the case of storage in methanol solution at room temperature for one month and two months. Acylcarnitine solutions were acquired by dissolving acylcarnitine-stable isotopes into methanol to concentrations listed in Table 3. The acylcarnitine-stable isotopes were measured under the following conditions. The acylcarnitine solutions were measured immediately before storage (prepared at the time of use) and after storage for predetermined periods, and the residual rate (%) was calculated from the acquired peak area ratio using Equation 2 described below. System: LC-20A series (manufactured by Shimadzu Corporation) and 4000 QTRAP (manufactured by AB Sciex)

<LC Conditions>

Mobile phase: mobile phase A, 0.5 % (v/v) formic acid aqueous solution
mobile phase B, 500 mmol/L ammonium formate aqueous solution: 500mmol/L
ammonium hydroxide (9:1)/methanol=1/9
Analysis time: 70 minutes
Elution method: A blend ratio of Eluent B was linearly increased or decreased under the following gradient conditions.

0 minutes (0 % Eluent B) → 5 minutes (0 % Eluent B)
5.1 minutes (0 % Eluent B) → 40 minutes (100 % Eluent B)
40 minutes (100 % Eluent B) → 55 minutes (100 % Eluent B)
55 minutes (100 % Eluent B) → 55.1 minutes (0 % Eluent B)
55.1 minutes (0 % Eluent B) → 70 minutes (0 % Eluent B)

Flow rate: 0.2 mL/min
LC column: Scherzo SS-C18, 3$\mu$m, 3.0×150 mm (manufactured by Imtact Corporation)

Column temperature: 50 °C
Sample injection amount: 5 $\mu$L

<MS Conditions>

Ionization mode: ESI (electrospray ionization)
Ion source: Turbo IonSpray
Scan type: MRM
Polarity: Positive
Q1/Q3 is described in Table 3.

Equation 2:

$$Residual\ rate\ (\%) =$$

$$\left( \frac{Peak\ area\ acquired\ from\ acylcarnitine\ solution\ after\ storage\ for\ predetermined\ period}{Peak\ area\ acquired\ from\ acylcarnitine\ solution\ immediately\ before\ storage} \right)$$

$$\times 100$$

[Table 3]

| Compound Name | Code | Q1 | Q3 | Sol. Conc. ($\mu$mol/L) | St. Pd. | Res. Rt. |
|---|---|---|---|---|---|---|
| $^2H_9$-Carnitine | C0 | 171 | 103 | 30.4 | 1M | 100.9% |
| | | | | | 2M | 100.2% |
| $^2H_3$-Acetylcarnitine | C2 | 207 | 85 | 3.8 | 1M | 99.4% |
| | | | | | 2M | 12.8% |
| $^2H_3$-propionylcarnitine | C3 | 221 | 85 | 2.3 | 1M | 97.4% |
| | | | | | 2M | 38.5% |
| $^2H_3$-Butyrylcarnitine | C4 | 235 | 85 | 1.5 | 1M | 101.7% |
| | | | | | 2M | 63.8% |
| $^2H_9$-Isovalerylcarnitine | C5 | 255 | 85 | 1.5 | 1M | 100.3% |
| | | | | | 2M | 99.4% |
| $^2H_3$-(3-Hydroxyisovaleryl)-carnitine | C5OH | 271 | 85 | 1.5 | 1M | 99.9% |
| | | | | | 2M | 97.9% |
| $^2H_3$-Glutarylcarnitine | C5DH | 285 | 85 | 1.5 | 1M | 99.3% |
| | | | | | 2M | 63.3% |
| $^2H_3$-Octanoylcarnitine | C8 | 291 | 85 | 1.5 | 1M | 103.3% |
| | | | | | 2M | 70.3% |
| $^2H_3$-Dodecanoylcarnitine | C12 | 319 | 85 | 3.0 | 1M | 103.1% |
| | | | | | 2M | 84.9% |
| $^2H_9$-Myristoylcarnitine | C14 | 375 | 85 | 3.0 | 1M | 103.9% |
| | | | | | 2M | 67.8% |
| $^2H_3$-Palmitoylcarnitine | C16 | 403 | 85 | 3.0 | 1M | 101.2% |
| | | | | | 2M | 62.6% |
| $^2H_3$-Octadecanoylcarnitine | C18 | 431 | 85 | 3.0 | 1M | 103.7% |
| | | | | | 2M | 69.6% |
| 1M: One Month<br>2M: Two Months<br>Sol. Conc.: Solution Concentration<br>St Pd.: Storage Period<br>Res. Rt: Residual Rate | | | | | | |

To predict the stability of acylcarnitine, the acetylcarnitine (C2) which the largest degrease in the residual rate was observed for in Table 3 was used as an index. A stability test was conducted by storing acetylcarnitine solutions at concentrations listed in Table 3 at four temperatures including a severe condition (45 °C, 22 °C, 4 °C, and-20 °C) to predict stable periods at -20 °C and 4 °C. With regard to samples subjected to the evaluation, it was calculated with this prediction method that storing at 45 °C for 24 hours corresponds to storing at 4 °C for approx. 3 months and storing at -20°C for approx. 5 years.

<Prediction Method>

Solvent: methanol
Evaluation temperatures: 45 °C, 22 °C, 4 °C, -20 °C
Prediction method: Arrhenius model

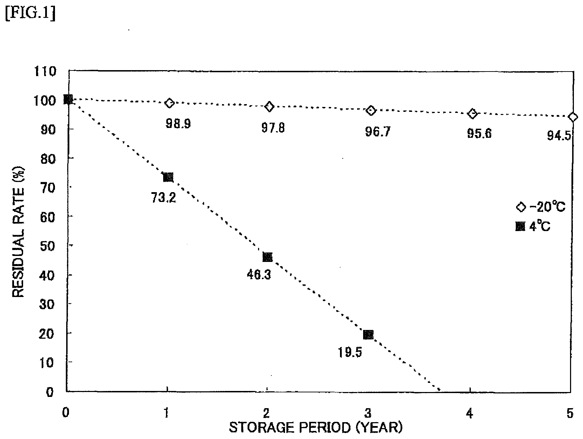

The acquired result is depicted in Fig. 1.

[Example 1] Stability of Acetylcarnitine (C2)-Stable Isotope in Methanol Containing 0.1 %(v/v) Formic Acid and Various Organic Solvents

Studies on dissolving solvents were conducted for acetylcarnitine (C2) which a largest decrease in the residual rate was recognized for in Reference Example 1 out of the selected acylcarnitines.

<Test Conditions>

Solvents: methanol, ethanol, isopropanol, acetonitrile, methanol containing 0.1 %(v/v) formic acid, 50 %(v/v) methanol aqueous solution
Storage temperature: 45 °C
Storage time: 24 hours

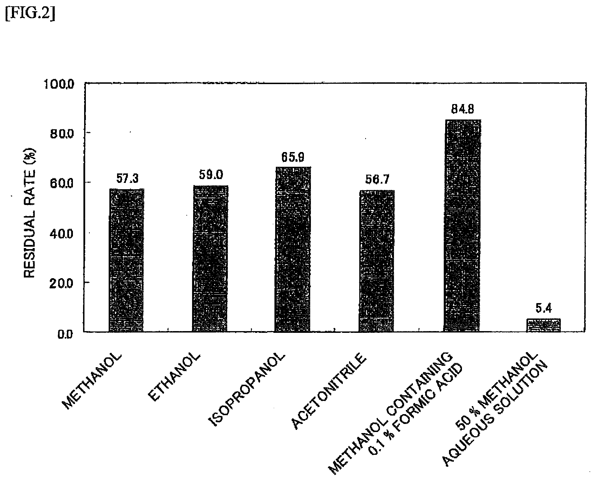

The measurement and the calculation of residual rate were performed in the same way as Reference Example 1. The acquired result is depicted in Fig. 2.

[Example 2] Stability of Acetylcarnitine (C2)-Stable Isotope in Methanol Containing 1 % Formic Acid (v/v) and Various Organic Solvents

As was the case with Example 1, studies were conducted with acetylcarnitine (C2) for confirming the effect of addition of an organic acid.

<Test Conditions>

Solvents: methanol, methanol containing 1 %(v/v) formic acid, ethanol containing 1 %(v/v) formic acid, isopropanol containing 1 %(v/v) formic acid, DMF
Storage temperature: 45 °C
Storage time: 24 hours

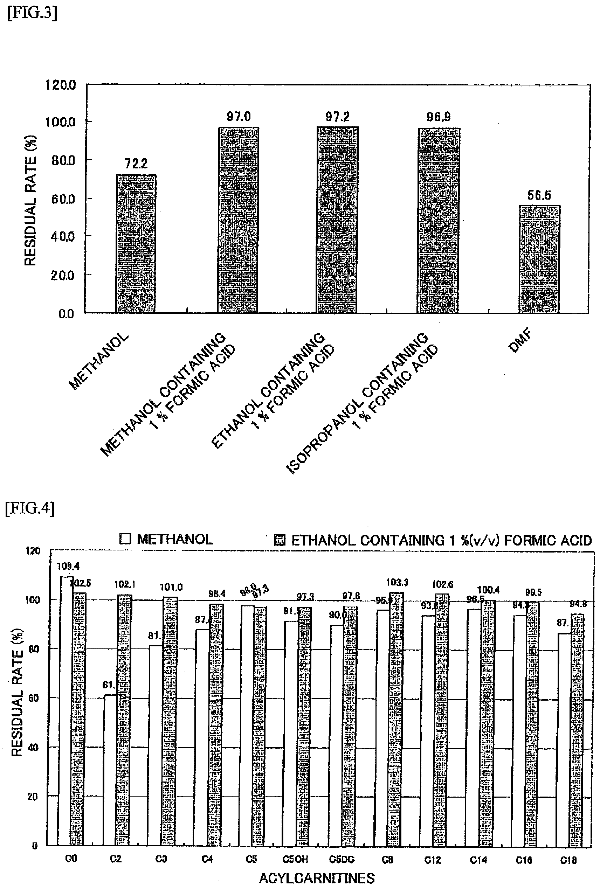

The measurement and the calculation of residual rate were performed in the same way as Reference Example 1. The acquired result is depicted in Fig. 3.

[Example 3] Stability of Acylcarnitine (C0 to C18)-Stable Isotopes in Ethanol Containing 1 %(v/v) Formic Acid and Methanol

Acylcarnitines (codes: C0, C2, C3, C4, C5, C5OH, C5DC, C8, C12, C14, C16, C18) shown in Table 3 were dissolved in ethanol containing 1 %(v/v) formic acid and methanol containing 1 %(v/v) formic acid studied in Example 2, and acylcarnitines other than acetylcarnitine (C2) were also examined in terms of storage stability.
An ethanol solution containing 1 %(v/v) formic acid and acylcarnitines was prepared as follows.

Acylcarnitines (codes: C0, C2, C3, C4, C5, C5OH, C5DC, C8, C12, C14, C16, and C18) were dissolved in ethanol containing a 1 %(v/v) formic acid to respective concentrations 15 times as high as the concentrations described in Table 3 to prepare ethanol solutions (12 solutions) containing 1 %(v/v) formic acid and acylcarnitines. Subsequently, 100 µL of each of the solutions were gathered and mixed, and 300 µL of ethanol containing 1 %(v/v) formic acid was added. The liquid mixture was well stirred to prepare 1500 µL of the 1 %(v/v) formic acid-containing ethanol solution containing each of the acylcarnitines at the concentrations described in Table 3.

For a methanol solution containing acylcarnitines, similarly, the acylcarnitines were dissolved in methanol to respective concentrations 15 times as high as the concentrations described in Table 3, and 100 µL of each of the solutions (12 solutions) were gathered and mixed before further adding 300 µL of methanol. The liquid mixture was well stirred to prepare 1500 µL of the methanol solution containing the acylcarnitines at the concentrations described in Table 3.

<Test Conditions>

Solvents: methanol, ethanol containing a 1 %(v/v) formic acid
Storage temperature: 45 °C
Storage time: 24 hours

The measurement and the calculation of residual rate were performed in the same way as Reference Example 1. The acquired result is depicted in Fig. 4.

Results and Discussion for Examples 1, 2, and 3

The result of Example 1 (Fig. 2) is as follows. While the storage stability of acetylcarnitine (C2) was lowest in the 50 %(v/v) methanol aqueous solution resulting in a residual rate of 5.4 %, improvements in the residual rate to 56.7 % to 65.9 % were observed in organic solvents (methanol, ethanol, isopropanol, and acetonitrile), and a significant improvement in the residual rate to 84.8 % was confirmed in methanol containing a 0.1 % formic acid.

In Example 2 (Fig. 3), the formic acid content of 0.1% in Example 1 is increased to 1 %. The formic acid was added not only to methanol but also to ethanol and isopropanol. As a result, the residual rate of acetylcarnitine was increased in accordance with the increase in the formic acid content, i.e., the residual rate was 84.8 % in methanol containing a 0.1 % formic acid (Example 1) and 97.0 % in methanol containing a 1 %(v/v) formic acid. The residual rates in ethanol containing a 1 % formic acid and isopropanol containing a 1 %(v/v) formic acid were 97.2 % and 96.9 %, respectively, and thus, were at the same level as the residual rate in methanol containing a 1 %(v/v) formic acid.

In Example 3 (Fig. 4), acylcarnitines (codes: C0, C2, C3, C4, C5, C5OH, C5DC, C8, C12, C14, C16, and C18) were dissolved in ethanol containing 1 %(v/v) formic acid to study differences in stability due to the chain length of fatty acid of acylcarnitine. As a result, the residual rates were 94.8 % to 103.3 %, and no difference in the residual rate was recognized from chain length differences of short-chain, medium-chain, and long-chain fatty acids.

From these results of Figs. 2, 3, and 4, with regard to the storage stability of acylcarnitine in solution, it has been revealed that acylcarnitine is more stable when stored in an organic solvent as compared to an aqueous solution and is more stable in an organic solvent containing an organic acid as compared to when it is dissolved in an organic solvent only. Additionally, it has been indicated that the storage stability of acylcarnitine is improved as the organic acid content increases. No significant difference was observed in the storage stability of acylcarnitine in an organic solvent containing an organic acid due to the chain length of fatty acid of acylcarnitine.

[Example 4] Storage Stability of Acetylcarnitine (C2)-Stable Isotope in Acetonitrile Containing 1 %(v/v) Formic Acid

As was the case with Example 1, the storage stability effect by the addition of an organic acid was confirmed for acetylcarnitine (C2) by using acetonitrile as the organic solvent. For comparison, acetonitrile, methanol, and ethanol, each alone, was also tested to confirm the storage stability effect.

<Test Conditions>

Solvents: methanol, ethanol, acetonitrile, and acetonitrile containing 1 %(v/v) formic acid
Storage temperature: 60 °C
Storage time: 24 hours

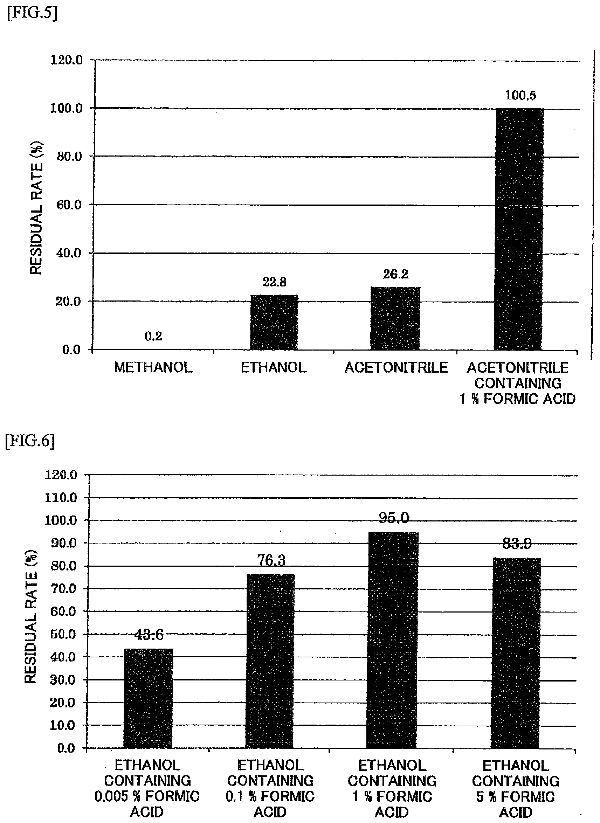

The measurement and the calculation of the residual rate were performed in the same way as Reference Example 1. The acquired result is depicted in Fig. 5.

[Example 5] Storage Stability of Acetylcarnitine (C2)-Stable Isotope in Ethanol Containing Formic Acid

As was the case with Example 1, the storage stability effect was confirmed for acetylcarnitine (C2) in solvents acquired by adding formic acid to ethanol at indicated concentrations.

<Test Conditions>

Solvents: ethanol containing formic acid at concentrations of 0.005 %, 0.1 %, 1 %, and 5 %(v/v)
Storage temperature: 60 °C
Storage time: 24 hours

The measurement and the calculation of the residual rate were performed in the same way as Reference Example 1. The acquired result is depicted in Fig. 6.

[Examples 6 to 9] Storage Stability of Acetylcarnitine (C2)-Stable Isotope in Ethanol Containing Organic Acid

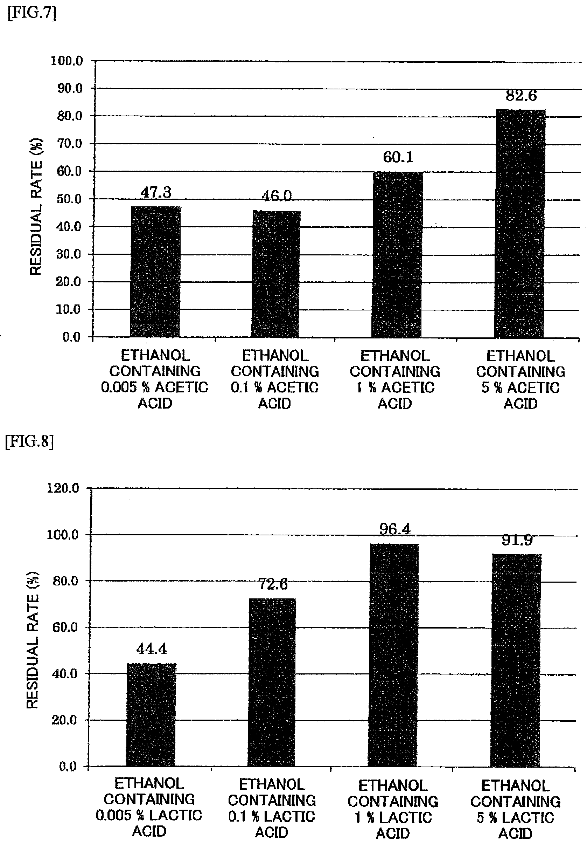

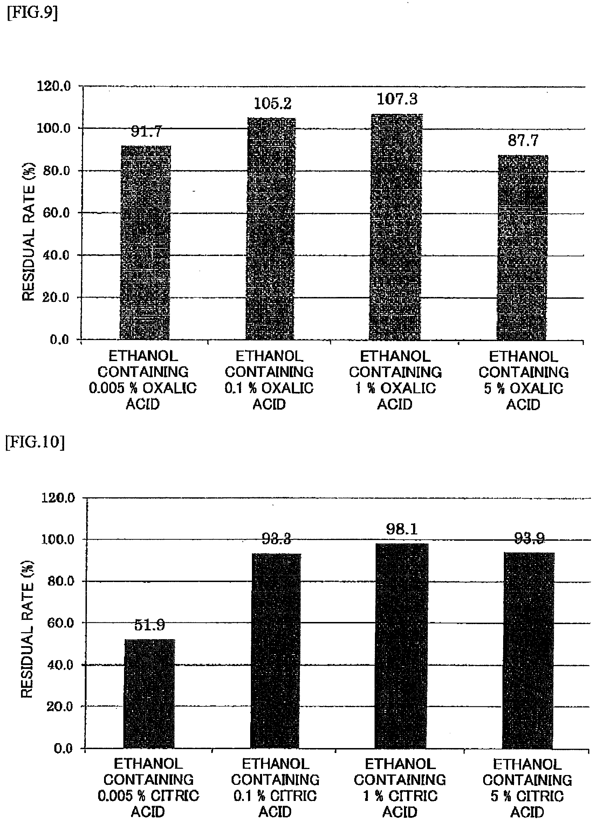

The storage stability effect was confirmed by conducting tests in the same way as Example 5 except that acetic acid, lactic acid, oxalic acid, and citric acid were used as the organic acid. Acquired results are depicted in Figs. 7 to 10.

Results and Discussion for Example 4

From the result of Example 4 (Fig. 5), although the storage stability of acetylcarnitine (C2) was low in each of the solely used organic solvents and was 26.2 % in acetonitrile, the residual rate was 100 % in acetonitrile containing 1 %(v/v) formic acid acquired by adding the organic acid to acetonitrile even under the severe condition of 60 °C and 24 hours, and a significant improvement in residual rate by addition of an organic acid was confirmed.
From the above, the effect of addition of an organic acid was confirmed even when acetonitrile was used as the organic solvent.

Results and Discussion for Examples 5 to 9

From the results of Examples 5 to 9 (Figs. 6 to 10), the storage stability of acetylcarnitine (C2) was high when either of formic acid, acetic acid, lactic acid, oxalic acid, or citric acid was used as the organic acid and added to the organic solvent, and thus, the effect by the addition of various organic acids was confirmed. With regard to the addition concentration, an improvement in the storage stability by the addition of the organic acid was observed in a wide range of 0.005 % to 5 %.

INDUSTRIAL APPLICABILITY

The acylcarnitine composition according to the present invention can preferably be used in the measurement of acylcarnitine using a mass spectrometer as a standard substance, a calibrator, a control, an internal standard substance, or the like, and has a great industrial applicability.

**Claims**

1. An acylcarnitine composition, wherein
   at least one or more compounds of Formula 1 are dissolved in an organic solvent containing an organic acid:

   Formula 1            : $(CH_3)_3N^+CH_2CH(OR^1)CH_2COO^-$

   wherein in Formula 1, $R^1$ denotes a hydrogen atom, or a saturated or unsaturated fatty acid residue having a carbon number of 2 to 30, and wherein the hydrogen atom(s) bonded to the fatty acid residue may be substituted with an oxygen atom(s) or a hydroxy group(s).

2. The acylcarnitine composition according to claim 1, wherein the organic solvent is an organic solvent having a carbon number of 1 to 5.

3. The acylcarnitine composition according to claim 1 or 2, wherein the organic solvent is at least one or more selected from the group consisting of alcohol having a carbon number of 1 to 5 and acetonitrile.

4. The acylcarnitine composition according to claim 3, wherein the alcohol having a carbon number of 1 to 5 is at least one or more selected from the group consisting of methanol, ethanol, and isopropanol.

5. The acylcarnitine composition according to any one of claims 1 to 4, wherein the organic acid is at least one or more selected from the group consisting of monovalent to trivalent carboxylic acids.

6. The acylcarnitine composition according to claim 5, wherein the carboxylic acid is at least one or more selected from the group consisting of formic acid, acetic acid, oxalic acid, trifluoroacetic acid, citric acid, butyric acid, and lactic acid.

7. The acylcarnitine composition according to any one of claims 1 to 6, wherein the volumetric concentration of the organic acid is 0.005 %(v/v) to 5 %(v/v).

8. The acylcarnitine composition according to any one of claims 1 to 7, wherein acylcarnitine is isotope-labeled.

9. The acylcarnitine composition according to any one of claims 1 to 8, wherein when the acylcarnitine composition is stored at 45 °C for 24 hours, the residual rate of acylcarnitine calculated from the value of measurement with a mass spectrometer is 80 % to 120 %.

10. A method of measuring acylcarnitine with a mass spectrometer, the method comprising: using the acylcarnitine composition according to any one of claims 1 to 9.

11. A use of the acylcarnitine composition according to any one of claims 1 to 9 in a diagnosis and screening for inborn errors of metabolism with a mass spectrometer.

12. A kit comprising: the acylcarnitine composition according to any one of claims 1 to 9.

13. A kit comprising: an organic acid, an organic solvent, and at least one or more acylcarnitine compounds of Formula 1:

$$\text{Formula 1} \qquad : (CH_3)_3N^+CH_2CH(OR^1)CH_2COO^-$$

wherein in Formula 1, $R^1$ denotes a hydrogen atom, or a saturated or unsaturated fatty acid residue having a carbon number of 2 to 30, and wherein the hydrogen atom(s) bonded to the fatty acid residue may be substituted with an oxygen atom(s) or a hydroxy group(s).

[FIG.1]

[FIG.2]

[FIG.3]

[FIG.4]

[FIG.5]

[FIG.6]

[FIG.7]

[FIG.8]

[FIG.9]

[FIG.10]

**EP 3 127 895 A1**

## INTERNATIONAL SEARCH REPORT

| International application No. |
| --- |
| PCT/JP2015/060110 |

**A. CLASSIFICATION OF SUBJECT MATTER**
*C07C227/44*(2006.01)i, *C07C229/22*(2006.01)i, *G01N27/62*(2006.01)i, *G01N33/50*(2006.01)i

According to International Patent Classification (IPC) or to both national classification and IPC

**B. FIELDS SEARCHED**

Minimum documentation searched (classification system followed by classification symbols)
C07C227/44, C07C229/22, G01N27/62, G01N33/50

Documentation searched other than minimum documentation to the extent that such documents are included in the fields searched

| | | | | |
| --- | --- | --- | --- | --- |
| Jitsuyo Shinan Koho | 1922–1996 | Jitsuyo Shinan Toroku Koho | 1996–2015 |
| Kokai Jitsuyo Shinan Koho | 1971–2015 | Toroku Jitsuyo Shinan Koho | 1994–2015 |

Electronic data base consulted during the international search (name of data base and, where practicable, search terms used)
CAplus(STN)

**C. DOCUMENTS CONSIDERED TO BE RELEVANT**

| Category* | Citation of document, with indication, where appropriate, of the relevant passages | Relevant to claim No. |
| --- | --- | --- |
| X | CN 103278584 A (SHANGHAI CHILDREN'S HOSPITAL), 04 September 2013 (04.09.2013), abstract; claims; examples (Family: none) | 1–13 |
| X | WO 2013/157945 A1 (UNIVERSITEIT LEIDEN), 24 October 2013 (24.10.2013), claims; example 5 & EP 2838631 A1 | 1–7,9–11 |
| X | CN 102565251 A (BEIJING ORIENT BERLIN MEDICAL TECHNOLOGY CO., LTD.), 11 July 2012 (11.07.2012), abstract; claims (Family: none) | 1–7,9–11 |

☐ Further documents are listed in the continuation of Box C.    ☐ See patent family annex.

| | |
| --- | --- |
| * Special categories of cited documents: <br> "A" document defining the general state of the art which is not considered to be of particular relevance <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search <br> 11 June 2015 (11.06.15) | Date of mailing of the international search report <br> 23 June 2015 (23.06.15) |
| --- | --- |
| Name and mailing address of the ISA/ <br> Japan Patent Office <br> 3-4-3,Kasumigaseki,Chiyoda-ku, <br> Tokyo 100-8915,Japan | Authorized officer <br><br> Telephone No. |

Form PCT/ISA/210 (second sheet) (July 2009)

## EP 3 127 895 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4838129 B

**Non-patent literature cited in the description**

- 3040-0010 NeoBase Non-derivatized MSMS kit Instruction Manual (PerkinElmer)
- Basic Research Project for Promotion of Next Generation for Overcoming Developmental Disease. Stability of Acylcarnitine in Liquid Sample. Health and Labour Sciences Research Grants in FY, 2011
- Study on the Development and Qualitative Improvement of Mass-Screening System for Newborns through Introduction of Tandem Mass. **SEIJI YAMAGUCHI et al.** General/Allotted Research Report. Ministry of Health, Labor and Welfare, 2011, 40-43

21